# EUROPEAN PATENT APPLICATION

(11) **EP 1 704 822 A1**
(43) Date of publication of application: **27.09.2006**
(21) Application number: 06251579.6
(22) Date of filing: 23.03.2006
(51) Int. Cl.: A61B 17/00, A61B 1/31, A61M 25/01, A61B 17/34

(54) **Catheter-gripping device which measures insertion force during a medical procedure**

(30) Priority: 24.03.2005 US 89181
(71) Applicant: ETHICON ENDO-SURGERY, INC., Cincinnati, Ohio 45242 (US)
(72) Inventor: Bakos, Gregory J., Mason, Ohio 45040 (US); Long, Gary L., Cincinnati, Ohio 45227 (US)
(74) Representative: Fisher, Adrian John

(57) **Abstract**

A catheter-gripping device measures a catheter-insertion force during a medical procedure. A handle includes first and second handle members adapted to be held by a clinician and which are disposable in a closed-handle position to at least partially surround a medical catheter having a distal end insertable into a body lumen of a patient. The first slide subassembly is slidably attached to the first handle member and is constrained to be slidable with respect to the first handle member only along an axis substantially parallel to the lengthwise axis of the handle. The second slide subassembly is likewise attached to the second handle member. The two slide subassemblies together grip the catheter when the handle members are disposed in the closed-handle position. The load cell has a force-measuring axis aligned substantially parallel to the lengthwise axis and is operably attached to the first slide subassembly and the first handle member.

## Description

**Field of the Invention**

The present invention is related generally to medical catheters, and more particularly to a catheter-gripping device which measures an insertion force on the catheter applied by a clinician during a medical procedure.

**Background of the Invention**

Medical catheters include, without limitation, insertion tubes of endoscopes such as insertion tubes of gastroscopes and colonoscopes. Insertion of the tube of a flexible endoscope, especially into the colon, can be very time-consuming and uncomfortable procedure for the patient, even when sedated with drugs. A physician often needs several minutes to push the tube of the flexible endoscope through the convoluted sigmoid, descending, transverse, and ascending portions of the colon. The physician may diagnose and/or treat tissues within the colon either during insertion or removal of the endoscope tube. The flexible endoscope tube may "loop" within the colon, such as at the sigmoid colon or at the splenic flexure of the colon, so that it becomes difficult to further advance the endoscope tube along the colon. When a loop is formed, the force exerted to push the tube stretches the mesentery and causes pain for the patient.

A known device for measuring the force exerted by a physician on the tube of a flexible endoscope during a colonoscopy includes a handgrip which surrounds and locks onto the tube. An inner handgrip part is attached to an outer handgrip part through a transversely extending bar containing strain gauges from which the exertion force is measured.

Still, scientists and engineers continue to seek improved devices for measuring the insertion force applied to a catheter during a medical procedure.

**Summary**

A first expression of an embodiment of a catheter-gripping device, which measures a catheter-insertion force during a medical procedure, includes a handle, a first slide subassembly, a second slide subassembly, and a load cell. The handle has a lengthwise axis and includes first and second handle members which are adapted to be held by a medical clinician and which are positionable in a closed-handle position to at least partially surround a medical catheter having a distal end insertable into a body lumen of a patient. The first slide subassembly is slidably attached to the first handle member and is constrained to be slidable with respect to the first handle member only along an axis substantially parallel to the lengthwise axis. The second slide subassembly is slidably attached to the second handle member and is constrained to be slidable with respect to the second handle member only along an axis substantially parallel to the lengthwise axis. The first and second slide subassemblies together grip the catheter when the first and second handle members are disposed in the closed-handle position. The load cell has a force-measuring axis aligned substantially parallel to the lengthwise axis and is operably attached to the first slide subassembly and to the first handle member.

A second expression of an embodiment of a catheter-gripping device, which measures a catheter-insertion force during a medical procedure, includes a handle, a first slide subassembly, a second slide subassembly, a load cell, and a hinge assembly. The handle has a lengthwise axis and includes first and second handle members which are adapted to be held by a medical clinician and which are positionable in a closed-handle position to at least partially surround a medical catheter having a distal end insertable into a body lumen of a patient. The first slide subassembly is slidably attached to the first handle member and is constrained to be slidable with respect to the first handle member only along an axis substantially parallel to the lengthwise axis. The second slide subassembly is slidably attached to the second handle member and is constrained to be slidable with respect to the second handle member only along an axis substantially parallel to the lengthwise axis. The first and second slide subassemblies together grip the catheter when the first and second handle members are disposed in the closed-handle position. The load cell has a force-measuring axis aligned substantially parallel to the lengthwise axis and is operably attached to the first slide subassembly and to the first handle member. The hinge assembly hingeably connects together the first and second handle members and biases the first and second handle members to an open handle position when the first and second handle members are not being held in the closed handle position by the clinician.

A third expression of an embodiment of a catheter-gripping device, which measures a catheter-insertion force during a medical procedure, includes a handle, a first slide subassembly, a second slide subassembly, a load cell, and a hinge assembly. The handle has a lengthwise axis and includes first and second handle members which are adapted to be held by a medical clinician and which are positionable in a closed-handle position to at least partially surround a medical catheter having a distal end insertable into a body lumen of a patient. The first slide subassembly is slidably attached to the first handle member and is constrained to be slidable with respect to the first handle member only along an axis substantially parallel to the lengthwise axis. The first slide subassembly includes one of a first slider portion and a first carriage portion of a first roller slide. The second slide subassembly is slidably attached to the second handle member and is constrained to be slidable with respect to the second handle member only along an axis substantially parallel to the lengthwise axis. The second slide subassembly includes one of a second slider portion and a second carriage portion of a second roller slide. The first and second slide subassemblies together grip the catheter when the first and second handle members are disposed in the closed-handle position. The load cell has a force-measuring axis aligned substantially parallel to the lengthwise axis and is operably attached to the first slide subassembly and to the first handle member. The hinge assembly hingeably connects together the first and second handle members.
The present invention also provides a method for measuring a catheter-insertion force, using one of the previously described catheter-gripping devices or one as defined in any of the appended claims, comprising the steps of:
a) inserting the distal end of the catheter into the body lumen of the patient;
b) hingeably closing the first and second handle members to at least partially surround the catheter in the closed-handle position;
c) using the handle in the closed-handle position to push the catheter a further distance into the body lumen of the patient; and
d) measuring the catheter-insertion force exerted on the handle using an output signal from the load cell.

Several benefits and advantages are obtained from one or more of the expressions of an embodiment of the invention. In one application, having a load cell and slide arrangement, such as a roller slide arrangement, provides a more reliable and easier to make catheter insertion-force measuring device. In the same or a different application, having hingeably-connected first and second handle members biased to an open handle position provides a catheter insertion-force measuring device which is easier to use as the clinician relocates the handle to different locations along the length of the catheter at different times during the medical procedure.

**Brief Description of the Figures**

FIGURE 1 is a perspective view of an embodiment of a catheter-gripping device of the invention shown with the handle in the open-handle position;

FIGURE 2 is a hinge-side elevational view of the catheter-gripping device of Figure 1 shown with the handle in the closed-handle position;

FIGURE 3 is a longitudinal cross-sectional view of the catheter-gripping device of Figure 1 shown with the handle in the closed-handle position; and

FIGURE 4 is a front elevational view of the catheter-gripping device of Figure 1 shown with the handle in the closed-handle position and shown with the second elastomeric member omitted for clarity.

**Detailed Description**

Before explaining the several expressions of an embodiment of the present invention in detail, it should be noted that each expression is not limited in its application or use to the details of construction and arrangement of parts and steps illustrated in the accompanying drawings and description. The illustrative expressions of an embodiment of the invention may be implemented or incorporated in other expressions, embodiments, variations and modifications, and may be practiced or carried out in various ways. Furthermore, unless otherwise indicated, the terms and expressions employed herein have been chosen for the purpose of describing the illustrative embodiment of the present invention for the convenience of the reader and are not for the purpose of limiting the invention.

It is further understood that any one or more of the following-described expressions, examples, etc. can be combined with any one or more of the other following-described expressions, examples, etc.

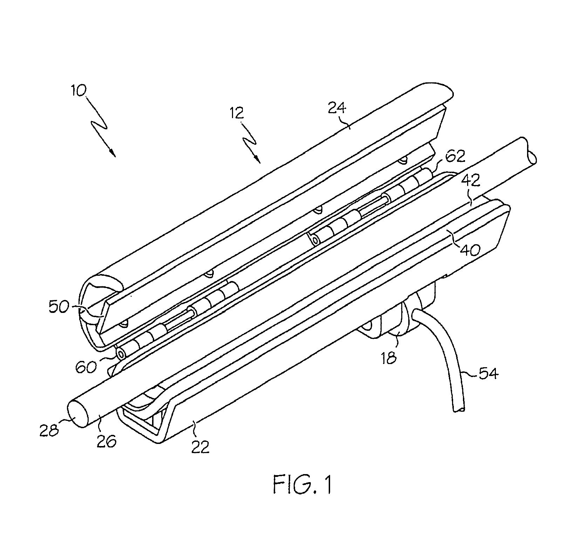

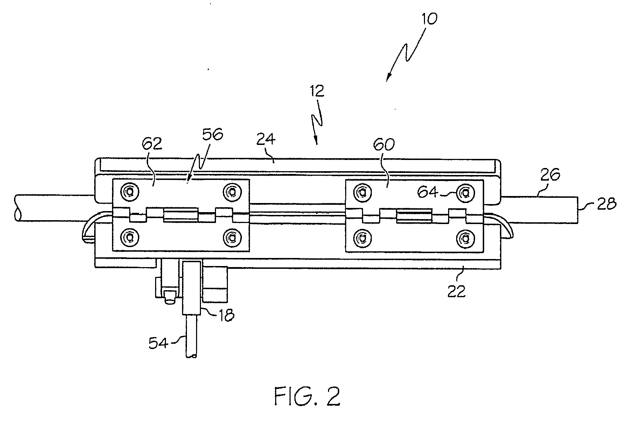

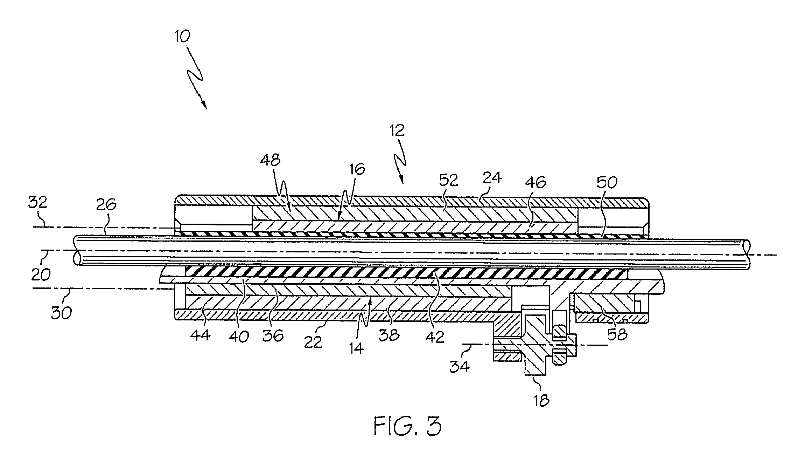

An embodiment of a catheter-gripping device 10 of the invention is shown in Figures 1-4. A first expression of the embodiment of Figures 1-4 is for a catheter-gripping device 10 including a handle 12, a first slide subassembly 14, a second slide subassembly 16, and a load cell 18. The handle 12 has a lengthwise axis 20 and includes first and second handle members 22 and 24 which are adapted to be held by a medical clinician and which are disposable in a closed-handle position (see especially Figures 3-4) to at least partially surround a medical catheter 26 having a distal end 28 insertable into a body lumen of a patient. The first slide subassembly 14 is slidably attached to the first handle member 22 and is constrained to be slidable with respect to the first handle member 22 only along an axis 30 substantially parallel to the lengthwise axis 20. The second slide subassembly 16 is slidably attached to the second handle member 24 and is constrained to be slidable with respect to the second handle member 24 only along an axis 32 substantially parallel to the lengthwise axis 20. The first and second slide subassemblies 14 and 16 together grip the catheter 26 when the first and second handle members 22 and 24 are disposed in the closed-handle position (see especially Figures 3-4). The load cell 18 has a force-measuring axis 34 aligned substantially parallel to the lengthwise axis 20 and is operably attached to the first slide subassembly 14 and to the first handle member 22.

Examples of medical catheters include, without limitation, cardiovascular catheters and insertion tubes of endoscopes such as insertion tubes of gastroscopes and colonoscopes. In one illustration of the catheter-gripping device 10 of the embodiment of Figures 1-4, the catheter 10 is an insertion tube of a flexible endoscope. Examples of body lumens of a patient include, without limitation, the upper GI (gastrointestinal) tract, the lower GI tract, and blood vessel passageways. Other examples of medical catheters 10 and/or body lumens are left to the artisan.

It is noted that the term "attached" includes directly attached and indirectly attached. For example, in one enablement of the first slide subassembly 14 shown in Figures 1 and 3-4, the first slide subassembly 14 includes a first carriage portion 36 of a first roller slide 38, a slider 40 non-slidably attached to the first carriage portion 36, and a first elastomeric member 42 non-slidably attached to the slider 40. It is noted that a roller slide is a well known mechanism which permits relative linear motion between the carriage portion and the slider portion of the roller slide with such motion constrained to be along an axis of the roller slide. In this enablement, the first slide subassembly 14 is indirectly attached to the first handle member 22 through a first slider portion 44 of the first roller slide 38, wherein the first slider portion 44 is not considered to be a part of the first slide subassembly 14 and is non-slidably attached to the first handle member 22 to make the first slide subassembly 14 slidably and indirectly attached to the first handle member 22. Non-slidable attachment techniques include, without limitation, using machine screws and adhesives, not shown.

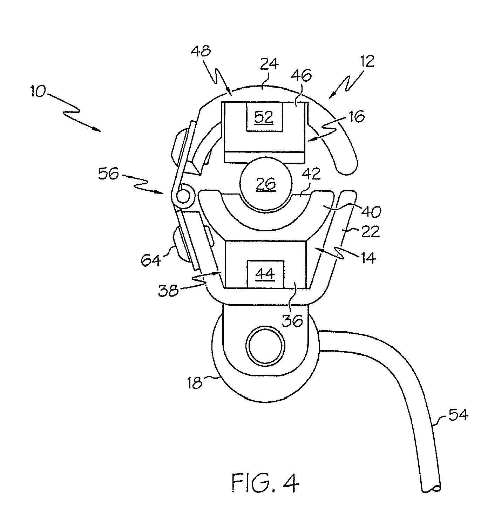

Similarly, in the enablement of the second slide subassembly 16 shown in Figures 1 and 3-4, the second slide subassembly 16 includes a second carriage portion 46 of a second roller slide 48 and includes a second elastomeric member 50 (seen in Figures 1 and 3 but omitted from Figure 4 for clarity) non-slidably attached to the second carriage portion 46. In this enablement, the second slide subassembly 16 is indirectly attached to the second handle member 24 through a second slider portion 52 of the second roller slide 48, wherein the second slider portion 52 is not considered to be a part of the second slide subassembly 16 and is non-slidably-attached to the second handle member 24 to make the second slide subassembly 16 slidably and indirectly attached to the second handle member 24.

In a different enablement, not shown, the first slide assembly is a monolithic member having a "T" rail portion slidably and directly attached to a matching "T" groove of the first handle member. Likewise, in one variation, the second slide assembly is a monolithic member having a "T" rail portion slidably and directly attached to a matching "T" groove of the second handle member. Other constructions of the first and/or second slide subassemblies 14 and 16 and other direct and/or indirect attachments are left to the artisan.

In one application of the first expression of the embodiment of Figures 1-4, the load cell 18 includes a cable 54 which carries an output signal from the load cell 18, and wherein the output signal corresponds to the catheter-insertion force exerted by the clinician on the handle 12 during the medical procedure. In one variation, not shown, the free end of the cable 54 is operably attached to a computer. In one modification, the computer includes a computer program which analyzes the output signal from the load cell 18 and displays the insertion force on a monitor as a function the position of the distal end 28 of the catheter 26 within a body lumen of a patient, such position being determined by other methods as is known to those skilled in the art. In one example, a level of pain experienced by the patient is determined and associated with the current measured insertion force, wherein, in this example, the level of pain is assumed to be related to the force exerted by the distal end 28 of the catheter 26 on the patient, and the force exerted by the distal end 28 of the catheter 26 on the patient is assumed to be related to the insertion force applied to the handle 12 by the clinician.

In one implementation of the first expression of the embodiment of Figures 1-4, the catheter 10 has a length, and the first and second handle members 22 and 24 are relocatable to at least partially surround the catheter 26 in the closed-handle position (see especially Figures 3-4) at different locations along the length of the catheter 26 at different times during the medical procedure. Such relocation is necessary for long catheters 10 as is known to the clinician. For example, when the catheter is an insertion tube of a flexible endoscope, the length of the catheter can exceed 1.5 meters.

In one arrangement of the first expression of the embodiment of Figures 1-4, the first and second slide subassemblies 14 and 16 are substantially diametrically aligned about the catheter 26 when the first and second handle members 22 and 24 are disposed in the closed-handle position (see especially Figures 3-4). Other arrangements involving other alignments are left to the artisan. Other constructions of the catheter-gripping device, not shown, involving two, three, or more handle members, are left to those skilled in the art.

A method for measuring a catheter-insertion force, using the catheter-gripping device 10 of the first expression of the embodiment of Figures 1-4, includes several steps. One step includes inserting the distal end 28 of the catheter 26 into the body lumen of the patient. Another step includes disposing the first and second handle members 22 and 24 to at least partially surround the catheter 26 in the closed-handle position. Another step includes using the handle 12 in the closed-handle position to push the catheter 26 a further distance into the body lumen of the patient. Another step includes measuring the catheter-insertion force exerted on the handle 12 using an output signal from the load cell 18.

A second expression of the embodiment of Figures 1-4 is for a catheter-gripping device 10 including a handle 12, a first slide subassembly 14, a second slide subassembly 16, a load cell 18, and a hinge assembly 56. The handle 12 has a lengthwise axis 20 and includes first and second handle members 22 and 24 which are adapted to be held by a medical clinician and which are disposable in a closed-handle position to at least partially surround a medical catheter 26 having a distal end 28 insertable into a body lumen of a patient. The first slide subassembly 14 is slidably attached to the first handle member 22 and is constrained to be slidable with respect to the first handle member 22 only along an axis 30 substantially parallel to the lengthwise axis 20. The second slide subassembly 16 is slidably attached to the second handle member 24 and is constrained to be slidable with respect to the second handle member 24 only along an axis 32 substantially parallel to the lengthwise axis 20. The first and second slide subassemblies 14 and 16 together grip the catheter 26 when the first and second handle members 22 and 24 are disposed in the closed-handle position. The load cell 18 has a force-measuring axis 34 aligned substantially parallel to the lengthwise axis 20 and is operably attached to the first slide subassembly 14 and to the first handle member 22. The hinge assembly 56 hingeably connects together the first and second handle members 22 and 24 and biases the first and second handle members 22 and 24 to an open handle position (see Figure 1) when the first and second handle members 22 and 24 are not being held in the closed handle position by the clinician.

It is noted that the illustrations, applications, arrangements, etc. of the first expression of the embodiment of Figures 1-4 are equally applicable to the second expression of the embodiment of Figures 1-4.

A method for measuring a catheter-insertion force, using the catheter-gripping device 10 of the second expression of the embodiment of Figures 1-4, includes several steps. One step includes inserting the distal end 28 of the catheter 26 into the body lumen of the patient. Another step includes hingeably closing the first and second handle members 22 and 24 to at least partially surround the catheter 26 in the closed-handle position. Another step includes using the handle 12 in the closed-handle position to push the catheter 26 a further distance into the body lumen of the patient. Another step includes measuring the catheter-insertion force exerted on the handle 12 using an output signal from the load cell 18.

A third expression of the embodiment of Figures 1-4 is for a catheter-gripping device 10 including a handle 12, a first slide subassembly 14, a second slide subassembly 16, a load cell 18, and a hinge assembly 56. The handle 12 has a lengthwise axis 20 and includes first and second handle members 22 and 24 which are adapted to be held by a medical clinician and which are disposable in a closed-handle position to at least partially surround a medical catheter 26 having a distal end 28 insertable into a body lumen of a patient. The first slide subassembly 14 is slidably attached to the first handle member 22 and is constrained to be slidable with respect to the first handle member 22 only along an axis 30 substantially parallel to the lengthwise axis 20. The first slide subassembly 14 includes one of a first slider portion 44 and a first carriage portion 36 of a first roller slide 38. The second slide subassembly 16 is slidably attached to the second handle member 24 and is constrained to be slidable with respect to the second handle member 24 only along an axis 32 substantially parallel to the lengthwise axis 20. The second slide subassembly 16 includes one of a second slider portion 52 and a second carriage portion 46 of a second roller slide 48. The first and second slide subassemblies 14 and 16 together grip the catheter 26 when the first and second handle members 22 and 24 are disposed in the closed-handle position. The load cell 18 has a force-measuring axis 34 aligned substantially parallel to the lengthwise axis 20 and is operably attached to the first slide subassembly 14 and to the first handle member 22. The hinge assembly 56 hingeably connects together the first and second handle members 22 and 24.

In one configuration of the third expression of the embodiment of Figures 1-4, the catheter-gripping device 10 also includes the other, of the first slider portion 44 and the first carriage portion 36 of the first roller slide 38 non-slidably attached to the first handle member 22, and further includes the other, of the second slider portion 52 and the second carriage portion 46 of the second roller slide 48, non-slidably attached to the second handle member 24. In one variation, the catheter-gripping device 10 also includes a semi-circular cylindrical slider 40 non-slidably attached to the one of the first slider portion 44 and the first carriage portion 36 of the first roller slide 38, and further includes a first elastomeric member 50 non-slidably attached to the slider 40, wherein the first elastomeric member 50 is in contact with the catheter 26 when the first and second handle members 22 and 24 at least partially surround the catheter 26 in the closed-handle position. In one modification, the catheter-gripping device 10 also includes a second elastomeric member 50 non-slidably attached to the one of the second slider portion 52 and the second carriage portion 46 of the second roller slide 48, wherein the second elastomeric member 50 is in contact with the catheter 26 when the first and second handle members 22 and 24 at least partially surround the catheter 26 in the closed-handle position. In one example, the catheter-gripping device 10 includes a third roller slide 58 as shown in Figure 3.

In one elaboration of the third expression of the embodiment of Figures 1-4, the hinge assembly 56 biases the first and second handle members 22 and 24 to an open handle position when the first and second handle members 22 and 24 are not being held in the closed handle position by the clinician. In one variation, the hinge assembly 56 includes two lengthwise-spaced-apart spring hinges 60 and 62 biasing the first and second handle members 22 and 24 to the open handle position. In one modification, machine screws 64 are used for the hinge-to-handle-member attachments. Other variations of hinge assemblies, including the number of hinges and biasing mechanisms, are left to the artisan.

It is noted that the illustrations, applications, arrangements, etc. of the first expression of the embodiment of Figures 1-4 are equally applicable to the second expression of the embodiment of Figures 1-4.

A method for measuring a catheter-insertion force, using the catheter-gripping device 10 of the third expression of the embodiment of Figures 1-4, includes several steps. One step includes inserting the distal end 28 of the catheter 26 into the body lumen of the patient. Another step includes hingeably closing the first and second handle members 22 and 24 to at least partially surround the catheter 26 in the closed-handle position. Another step includes using the handle 12 in the closed-handle position to push the catheter 26 a further distance into the body lumen of the patient. Another step includes measuring the catheter-insertion force exerted on the handle 12 using an output signal from the load cell 18.

Several benefits and advantages are obtained from one or more of the expressions of an embodiment of the invention. In one application, having a load cell and slide arrangement, such as a roller slide arrangement, provides a more reliable and easier to make catheter insertion-force measuring device. In the same or a different application, having hingeably-connected first and second handle members biased to an open handle position provides a catheter insertion-force measuring device which is easier to use as the clinician relocates the handle to different locations along the length of the catheter at different times during the medical procedure.

While the present invention has been illustrated by a description of several expressions of an embodiment and examples, etc. thereof, it is not the intention of the applicants to restrict or limit the spirit and scope of the appended claims to such detail. Numerous other variations, changes, and substitutions will occur to those skilled in the art without departing from the scope of the invention. It will be understood that the foregoing description is provided by way of example, and that other modifications may occur to those skilled in the art without departing from the scope of the appended Claims.

## Claims

1. A catheter-gripping device, which measures a catheter-insertion force during a medical procedure, comprising:
a) a handle having a lengthwise axis and including first and second handle members which are adapted to be held by a medical clinician and which are disposable in a closed-handle position to at least partially surround a medical catheter having a distal end insertable into a body lumen of a patient;
b) a first slide subassembly slidably attached to the first handle member and constrained to be slidable with respect to the first handle member only along an axis substantially parallel to the lengthwise axis;
c) a second slide subassembly slidably attached to the second handle member and constrained to be slidable with respect to the second handle member only along an axis substantially parallel to the lengthwise axis, wherein the first and second slide subassemblies together grip the catheter when the first and second handle members are disposed in the closed-handle position; and
d) a load cell having a force-measuring axis aligned substantially parallel to the lengthwise axis and operably attached to the first slide subassembly and to the first handle member.

2. The catheter-gripping device of claim 1, wherein the catheter has a length, and wherein the first and second handle members are relocatable to at least partially surround the catheter in the closed-handle position at different locations along the length of the catheter at different times during the medical procedure.

3. The catheter-gripping device of claim 1 or claim 2, wherein the first and second slide subassemblies are substantially diametrically aligned about the catheter when the first and second handle members are disposed in the closed-handle position.

4. A catheter-gripping device, which measures a catheter-insertion force during a medical procedure, comprising:
a) a handle having a lengthwise axis and including first and second handle members which are adapted to be held by a medical clinician and which are disposable in a closed-handle position to at least partially surround a medical catheter having a distal end insertable into a body lumen of a patient;
b) a first slide subassembly slidably attached to the first handle member and constrained to be slidable with respect to the first handle member only along an axis substantially parallel to the lengthwise axis;
c) a second slide subassembly slidably attached to the second handle member and constrained to be slidable with respect to the second handle member only along an axis substantially parallel to the lengthwise axis, wherein the first and second slide subassemblies together grip the catheter when the first and second handle members are disposed in the closed-handle position;
d) a load cell having a force-measuring axis aligned substantially parallel to the lengthwise axis and operably attached to the first slide subassembly and to the first handle member; and
f) a hinge assembly hingeably connecting together the first and second handle members and biasing the first and second handle members to an open handle position when the first and second handle members are not being held in the closed handle position by the clinician.

5. A catheter-gripping device, which measures a catheter-insertion force during a medical procedure, comprising:
a) a handle having a lengthwise axis and including first and second handle members which are adapted to be held by a medical clinician and which are disposable in a closed-handle position to at least partially surround a medical catheter having a distal end insertable into a body lumen of a patient;
b) a first slide subassembly slidably attached to the first handle member and constrained to be slidable with respect to the first handle member only along an axis substantially parallel to the lengthwise axis, wherein the first slide subassembly includes one of a first slider portion and a first carriage portion of a first roller slide;
c) a second slide subassembly slidably attached to the second handle member and constrained to be slidable with respect to the second handle member only along an axis substantially parallel to the lengthwise axis, wherein the second slide subassembly includes one of a second slider portion and a second carriage portion of a second roller slide, and wherein the first and second slide subassemblies together grip the catheter when the first and second handle members are disposed in the closed-handle position;
d) a load cell having a force-measuring axis aligned substantially parallel to the lengthwise axis and operably attached to the first slide subassembly and to the first handle member; and
f) a hinge assembly hingeably connecting together the first and second handle members.

6. The catheter-gripping device of claim 5, also including the other, of the first slider portion and the first carriage portion of the first roller slide, non-slidably attached to the first handle member, and further including the other, of the second slider portion and the second carriage portion of the second roller slide, non-slidably attached to the second handle member.

7. The catheter-gripping device of claim 6, also including a semi-circular cylindrical slider non-slidably attached to the one of the first slider portion and the first carriage portion of the first roller slide, and further including a first elastomeric member non-slidably attached to the slider, wherein the first elastomeric member is in contact with the catheter when the first and second handle members at least partially surround the catheter in the closed-handle position.

8. The catheter-gripping device of claim 7, also including a second elastomeric member non-slidably attached to the one of the second slider portion and the second carriage portion of the second roller slide, wherein the second elastomeric member is in contact with the catheter when the first and second handle members at least partially surround the catheter in the closed-handle position.

9. The catheter-gripping device of any one of claims 5 to 8, wherein the hinge assembly biases the first and second handle members to an open handle position when the first and second handle members are not being held in the closed handle position by the clinician.

10. The catheter-gripping device of any one of claims 4 to 9, wherein the first and second slide subassemblies are substantially diametrically aligned about the catheter when the first and second handle members are disposed to at least partially surround the catheter.

11. The catheter-gripping device of any one of the preceding claims, wherein the catheter is an insertion tube of a flexible endoscope.

12. The catheter-gripping device of any one of the preceding claims, wherein the load cell includes a cable which carries an output signal from the load cell, and wherein the output signal corresponds to the catheter-insertion force exerted by the clinician on the handle during the medical procedure.

13. A method for measuring a catheter-insertion force, using the catheter-gripping device of claim 1, comprising the steps of:
a) inserting the distal end of the catheter into the body lumen of the patient;
b) disposing the first and second handle members to at least partially surround the catheter in the closed-handle position;
c) using the handle in the closed-handle position to push the catheter a further distance into the body lumen of the patient; and
d) measuring the catheter-insertion force exerted on the handle using an output signal from the load cell.
